(19) Europäisches Patentamt
European Patent Office
Office européen des brevets

(11) Publication number: **0 082 692**
**B1**

(12) **EUROPEAN PATENT SPECIFICATION**

(45) Date of publication of patent specification: **27.01.88**

(21) Application number: **82306754.1**

(22) Date of filing: **17.12.82**

(51) Int. Cl.[4]: **B 01 J 23/89,** B 01 J 27/24, C 07 C 1/04, C 07 C 27/20, C 07 C 29/15, C 07 C 29/14, C 07 C 29/136, C 07 C 51/10

(54) **Process for upgrading synthesis gas and catalysts therefor.**

(30) Priority: **21.12.81 US 332771**
**21.12.81 US 332772**

(43) Date of publication of application:
**29.06.83 Bulletin 83/26**

(45) Publication of the grant of the patent:
**27.01.88 Bulletin 88/04**

(84) Designated Contracting States:
**BE DE FR GB IT NL**

(56) References cited:
**EP-A-0 018 763**
**FR-A-2 231 416**
**FR-A-2 388 781**
**GB-A- 825 762**
**GB-A-1 219 281**
**GB-A-2 074 164**
**US-A-1 831 179**
**US-A-2 779 802**
**US-A-3 939 205**
**US-A-3 953 363**
**US-A-4 085 157**
**US-A-4 206 134**

(73) Proprietor: **THE STANDARD OIL COMPANY**
**Midland Building**
**Cleveland, Ohio 44115 (US)**

(72) Inventor: **Pesa, Frederick Anthony**
**764 Circlewood Drive**
**Aurora Ohio 44202 (US)**
Inventor: **Graham, Anne Marie**
**7304 Honeydale Drive**
**Northfield Center Ohio 44067 (US)**

(74) Representative: **Smith, Sydney et al**
**Elkington and Fife High Holborn House 52/54**
**High Holborn**
**London WC1V 6SH (GB)**

(56) References cited:
**US-A-4 235 798**
**US-A-4 261 927**

**EP 0 082 692 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention is directed to the upgrading of synthesis gas to produce mixtures of hydrocarbons and to catalysts of use in such process.

In particular, the present invention is directed to a vapour phase reaction of synthesis gas comprising carbon monoxide and hydrogen in the presence of a catalyst to produce mixtures of hydrocarbon and oxygenated hydrocarbons. It also relates to catalysts of use in such process.

U.S. Patent No. 2,476,788 discloses the synthesis of hydrocarbons, including oxygenates such as aldehydes, ketones and alcohols from carbon monoxide and hydrogen in the presence of metals or metal oxides selected from nickel, iron or cobalt, optionally with promoter metals or metal compounds of aluminium, cerium, magnesium, manganese, thorium, titanium, uranium, zinc, and zirconium. The catalyst could be supported on suitable carriers such as clay, silica gel, and alumina.

U.S. Patent Nos. 2,535,060 and 2,549,470 disclose the preparation of straight-chain primary hydroxyalkanes by introducing hydrogen, carbon monoxide and a hydroxylated solvent into a reaction vessel and heating the mixture in the presence of a ruthenium-containing catalyst (particularly ruthenium metal, oxide, carbonyl, or salts of carboxylic acids which give rise to formation of the carbonyl) and in Patent 2,549,470 in the presence of an alkaline reagent by maintaining pH in the range of 7.0 to 11.5. Both Patents teach that it is essential that the reaction take place in the liquid phase.

U.S. Patent No. 3,941,819 describes the production of ethane, ethylene and dimethyl ether by passing a carbon monoxide and hydrogen mixture over platinum supported on alumina.

U.S. Patent No. 4,014,913 discloses the preparation of acetic acid, ethanol and acetaldehyde by contacting $H_2$ and CO with a rhodium-manganese catalyst.

U.S. Patent No. 4,086,262 describes the production of hydrocarbon mixtures by contacting a mixture of carbon monoxide and hydrogen with a carbon monoxide reduction catalyst and an acidic crystalline alumino silicate (zeolite). Chang et al. teach that prominent types of catalysts include metals or oxides of Zn, Fe, Co, Ni, Ru, Th, Rh, and Os, and that "with the exception of ruthenium, all practical art recognized synthesis catalysts contain chemical and structural promoters".

U.S. Patent No. 4,096,164 discloses the production of oxygenated 2 carbon atom hydrocarbons by reacting CO and $H_2$ in the presence of catalysts comprising Rh, Mo and W.

U.S. Patent No. 4,101,460 and U.S. Patent No. 4,136,104 disclose the conversion of synthesis gas to acetic acid and related 2 carbon atom oxygenated derivatives in the presence of a rhodium metal/ruthenium metal catalyst.

U.S. Patent No. 4,116,994 discloses the selective production of olefinic hydrocarbons from carbon monoxide and hydrogen using a catalyst comprising rhodium deposited on titanium containing oxides.

U.S. Patent No. 4,119,656 describes the production of one to 2 carbon atom oxygenated hydrocarbons by contacting synthesis gas with a catalyst consisting essentially of palladium.

U.S. Patent No. 4,122,110 discloses the manufacture of linear saturated primary alcohols from synthesis gas using a catalyst comprising copper, cobalt, a third metal selected from chromium, iron, vanadium and manganese, at least one alkali metal and optionally zinc.

U.S. Patent No. 4,162,262 discloses the production of 2 carbon atom oxygenated hydrocarbons while minimizing co-production of methanol by reacting $H_2$ and CO with a catalyst containing rhodium metal, uranium or thorium and optionally iron, molybdenum or tungsten.

U.S. Patent No. 4,171,320 discloses the selective production of olefins from carbon monoxide and hydrogen using as a catalyst ruthenium on a support comprising at least one refractory Group VB metal oxide.

U.S. Patent No. 4,199,522 discloses the preparation of olefins of 2 to 4 carbon atoms from carbon monoxide and hydrogen using catalysts comprising a sulfide, oxide or metal of Mo, W, Re, Ru, Ni, Pd, Rh, Os, Ir or Pt and a hydroxide, oxide or salt of Li, Na, K, Rb, Cs, Mg, Ca, Sr, Ba or Th.

U.S. Patent No. 4,201, 597 discloses the preparation of oxygenated hydrocarbons by reacting carbon monoxide and hydrogen in the presence of a catalyst containing rhodium, tungsten and an alkali metal.

U.S. Patent No. 4,206,134 discloses the selective preparation of low weight olefins from carbon monoxide and hydrogen using as a catalyst ruthenium on a support consisting of a manganese-containing oxide.

U.S. Patent No. 4,235,801 discloses the preparation of ethanol by contacting a synthesis gas mixture containing CO and $H_2$ with a rhodium-iron catalyst.

U.S. Patent No. 4,246,186 discloses the preparation of two carbon atom oxygenated hydrocarbons from hydrogen and carbon monoxide by reaction with a rhodium metal catalyst, as compared to other single element Group VIII metal and copper catalysts.

European Patent Appln. No. 18,763 describes the production of oxygenated hydrocarbons having 1 to 4 carbon atoms by reaction of CO and $H_2$ in the presence of a catalyst comprising rhodium, chromium and optionally Fe, Mn, Mo, W or Ru. The catalyst may be prepared upon a support which has been formerly activated by the addition of metals or non-metals such as alkalis, Th, Mn, Rh, Fe, Cr, Mo, B, and P.

European Patent Appln. Nos. 4,653 and 4,656 describe the production of acetic acid, ethanol and acetaldehyde by reacting CO and $H_2$ with a catalyst containing rhodium, magnesium and a halide.

We have found that catalyst comprising the mixed oxides of ruthenium, copper and an alkali metal,

optionally promoted with an oxide of at least one of Ce, Cr, Fe, Mn, Mo, Zn or mixtures thereof, said catalysts being optionally nitrided, are useful for the upgrading of synthesis gas to hydrocarbons, particularly olefins, and oxygenated hydrocarbon products, particularly carboxylic acids, with high selectivity.

The invention provides such catalysts and processes for upgrading synthesis gas utilizing such catalysts.

In one embodiment, the process of the present invention includes the upgrading of synthesis gas to obtain selectivity to olefins and carboxylic acids comprising contacting carbon monoxide and hydrogen in the vapour phase at a reaction temperature of at least 250°C and a reaction pressure of at least 500 psi (3.447 MPa) with a catalyst of the formula

$$M_aA_bRuCu_cN_zO_x$$

wherein A is an alkali metal or an alkaline earth metal or mixtures thereof,
wherein M is Ce, Cr, Fe, Mn, Mo, Zn or mixtures thereof, and
wherein a is 0 to about 0.5
b is about 0.002 to about 2,
c is about 0.5 to about 3,
z is 0 to about 1 weight percent and
x is the number of oxygens needed to fulfill the valence requirements of the other elements.

The present invention further includes novel catalysts of the composition

$$M_aA_bRuCu_cN_zO_x$$

wherein A is an alkali metal or an alkaline earth metal or mixtures thereof,
wherein M is Ce, Cr, Fe, Mn, Mo, Zn or mixtures thereof, and
wherein a is 0 to about 0.5,
b is about 0.002 to about 2,
c is about 0.5 to about 3,
z is 0 to about 1 weight percent and
x is the number of oxygens needed to fulfill the valence requirements of the other elements.

The present invention further includes the upgrading of synthesis gas to yield hydrocarbons, namely alkanes, alcohols and esters useful for fuels, comprising:

contacting carbon monoxide and hydrogen in the vapour phase at a temperature of at least 250°C and a pressure of at least 500 psi (3.447 MPa) in the presence of a catalyst of the formula

$$M_aA_bRuCu_cN_zO_x$$

wherein A is an alkali metal or an alkaline earth metal or mixtures thereof,
wherein M is Ce, Cr, Fe, Mn, Mo, Zn or mixtures thereof, and
wherein a is 0 to about 0.5,
b is about 0.002 to about 2,
c is about 0.5 to about 3,
z is 0 to about 1 weight percent, and
x is the number of oxygens needed to fulfill the valence requirements of the other elements.

recovering the hydrocarbon and oxygenated hydrocarbon products;

contacting said products with hydrogen at elevated temperature and pressure in the presence of a hydrogenation catalyst.

Thus, in the process of the present invention, synthesis gas, or a mixture of carbon monoxide and hydrogen, is reacted in the presence of a carbon monoxide hydrogenation catalyst in the vapour phase to form hydrocarbons, and in particular, olefins and carboxylic acids in one embodiment of the invention, and alkanes and alcohols in another embodiment of the invention.

Synthesis gas may be produced by means known in the art and practiced commercially, including providing synthesis gas as a product of the partial combustion of coal, natural gas, petroleum bottoms or other carbonaceous materials. One method of derivation is the heating of coke in the presence of air and then steam. The ratio of carbon monoxide to hydrogen in the synthesis gas mixture to be upgraded may vary from about 1:10 to 10:1 and is preferably in the range of about 1:3 to about 3:1. The synthesis gas may contain a very low amount of sulfur compounds, and may also contain small amounts of carbon dioxide, nitrogen and other inerts.

Although synthesis gas is a preferred reactant, any other gas composed primarily of hydrogen and carbon monoxide and having $CO:H_2$ ratio of 1:10 to 10:1 may be employed. Preferably the gaseous reactant is essentially sulfur free.

Process conditions

The process of the present invention is carried out by contacting the gaseous reactants containing

carbon monoxide and hydrogen, with the novel catalyst described below in a suitable fluid bed or fixed bed reactor. The reaction can be conducted continuously or in a batch-type operation. The reaction temperature should be maintained between about 250°C to about 400°C, preferably 275°C to about 375°C.

The reaction pressure should normally be maintained between about 500 psi (3.447 MPa) to about 5000 psi (34.47 MPa) preferably 500 psi (3.447 MPa) to about 1500 psi (10.34 MPa). The reactant gases may be fed to the reactor utilized at a space velocity (litres gaseous reactants fed per litre of catalyst per hour) of about 100 per hour to about 10,000 per hour, preferably about 500 per hour to 6,000 per hour.

The contact time of the reactants with the catalyst is generally between about 10 seconds to about 200 seconds, and is preferably about 15 seconds to about 100 seconds.

## Catalyst

The novel catalyst provided in one embodiment of the present invention is believed to be an oxide complex and comprises the composition described by the empirical formula

$$M_aA_bRuCu_cN_zO_x$$

wherein A is an alkali metal or an alkaline earth metal or mixtures thereof,
wherein M is Ce, Cr, Fe, Mn, Mo, Zn or mixtures thereof, and
wherein a is 0 to about 0,5,
b is about 0.002 to about 2,
c is about 0.5 to about 3,
z is about 1 weight percent, and
x is the number of oxygens needed to fulfill the valence requirements of the other elements.

A may be selected from Na, Li, K, Rb, Cs, Be, Ms, Ca, Sr and Ba although Na, Li, Rb, Cs and Mg are preferred.

The ratio of ruthenium to copper is preferably about 0.5:1 to about 2:1. An oxide of alkali metal or an alkaline earth metal, preferably an alkali metal, is required in the present catalyst. Mixed oxide catalysts of ruthenium and copper which are alkali metal and alkaline earth metal free produce essentially all methane. The alkali metal or alkaline earth metal may be present in the catalyst at a level of about 0.002 to about 2 moles per mole of ruthenium oxide, most preferably about 0.02 to about 0.4. Preferred is a level of about 0.02 to about 1 mole alkali metal per mole of ruthenium oxide. The level of alkaline earth metal, if present, to ruthenium oxide is preferably 0.02 to about 0.5 moles per moles of ruthenium oxide. Preferably the M promoter is present in a level of 0.1 to 0.5 moles per mole of ruthenium oxide.

The catalyst of the present invention is a mixed metal oxide. In the process of the present invention, the catalyst is preferably utilized in a partially reduced state, however, the catalyst is not totally reduced to elemental metal and thus retains its oxide character.

The catalyst may be prepared by conventional means, such as mixing compounds containing the catalyst components in a liquid solution or slurry, such as a water solution or slurry and heating, recovering the catalyst precursor from the liquid, drying and calcining. Suitable catalyst component containing compounds may include but are not limited to oxides, hydroxides, inorganic salts such as nitrates, phosphates, halides, carbonates, silicates, aluminates, and salts of organic acids such as acetates, formates, butyrates, propionates, benzylates, and the like. Preferred catalysts of the present invention, containing the alkali metal component are prepared by recovering the catalyst precursor by adding to the aqueous solution of ruthenium, copper and promoter (if any) components, an alkali metal hydroxide to cause precipitation of the catalyst precursor, heating in the presence of the alkali metal, and thereafter filtering the precipitate.

The catalyst may be formed in a conventional manner, such as tableting, pelleting, or supporting the active catalyst material on a carrier. The carrier is preferably inert, and may include silica, alumina, Alundum (Registered Trade Mark), clay, alumina-silica, silicon carbide and the like. The active catalytic material may be coated on the carrier by the method described in U.S. Patent No. 4,077,912 or may be impregnated on the carrier such as by depositing a solution of the catalyst component containing compounds onto a carrier, drying and calcining. Catalyst components may be added to the carrier separately, if desired.

## Products

Products of the synthesis gas upgrading process of the present invention in which the M promoter metal is utilized in the catalyst include methane, gaseous alkanes, having more than one carbon atom and olefins having from 2 carbon atoms to about 4 carbon atoms; alcohols, carboxylic acids and aldehydes having from one to 5 carbon atoms present in an aqueous product phase; and olefins, carboxylic acids, esters, aldehydes and alcohols in an organic or oil product phase. Generally, very low amounts of higher weight paraffins are produced. The predominant products, however, are olefins and carboxylic acids.

Products of the synthesis gas upgrading process include, among others, methane, ethane, propane, butane, ethylene, propylene, butylene, methanol, ethanol, propanol, butanol, pentanol, acetic acid, propanoic acid, butyric acid, valeric acid, and low amounts of aldehydes and esters including acetaldehyde and methyl butyrate. These products are useful as chemical feedstocks, or as fuels, such as in gasoline

mixtures. Where conversion is maintained at a moderate or low level, these products can be recovered from the reactor effluent, and the remaining synthesis gas recycled to the reaction.

Alkanes, esters and alcohols are most suitable for use as fuels, such as in gasoline mixtures. Therefore, in one embodiment of the invention, the liquid product mixture obtained from the synthesis gas upgrading process (containing in addition to alcohols and esters, the non-fuel components such as olefins, aldehydes and carboxylic acids) is contacted with hydrogen at elevated temperature and pressure in the presence of a hydrogenation catalyst. The resulting hydrogenation products, alkanes, alcohols and esters are suitable for use as fuel components.

The hydrogenation process may be conducted in the vapour phase, at a reaction temperature of about 150°C to about 450°C and a reaction pressure of about 250 psig (1.723 MPa) to about 5000 psig (34.47 MPa). Any suitable hydrogenation catalyst, such as nickel or copper chromite may be used, although catalysts represented by the formula:

$$G_eRu_fD_gE_hO_x$$

are preferred,

(wherein G=Zn, Cd and mixtures thereof;
D=Co, Ni and mixtures thereof;
E=Fe, Cu, Rh, Pd, Os, Ir, Pt and mixtures thereof; and
wherein e=0 to 1,
f=0.01 to 3,
g=0.01 to 3,
h=0 to 1,
x=the number of oxygens determined by the valence requirements of the other elements).

The following Examples (1—36) are given by way of illustration only. In these Examples the following general procedures were followed:—

Catalyst preparation

In the examples below, catalysts were prepared by the following method. An amount of ruthenium chloride and copper chloride required to give 0.03 moles of each metal were dissolved in 250 millilitres of water with stirring for 30 minutes. Aqueous sodium hydroxide (50% by weight) was added dropwise, with stirring, until the pH reached and remained at 8.3 to 8.5 (approximately 7 to 15 millilitres). The resulting slurry was heated near boiling for 30 minutes with constant stirring, then cooled. The pH was adjusted if necessary to 7.5. The mixture was filtered, washed, and reslurried with subsequent filtering and washing steps until the molar ratio of sodium to ruthenium present was approximately 0.02 to 0.2:1. The solid mixed oxide was dried at 125°C for about 16 hours, was calcined for three hours at about 350°C (in air) and was ground to pass 140 mesh (0.105 millimeters).

The catalysts were coated upon alumina-silica supports in the following manner. 25 grams of Norton SA 5223 Alundum, 10/30 mesh (0.595 millimeters—2.00 millimeters) were placed in a vessel. 1.25 g distilled water was sprayed onto the Alundum which was rolled for approximately 10 minutes and the procedure was repeated. The metal oxide catalysts, in an amount calculated to give a total of 0.015 moles of active metal, was added in two equal portions with 15 minutes rolling after each. The coated catalyst was dried for about 16 hours at 125°C and calcined three hours at 350°C. Catalysts prepared in this manner contain approximately 5 weight percent active metals, 0.01% to 0.1% by weight sodium and have surface areas of about 2 $m^2/g$, with pore volumes of from about 0.06 to about 0.09 cc/g. Promoter elements were added either before precipitation of the RuCu precursor, or the RuCu oxide containing powder was impregnated with a solution of the promoter compound.

The catalysts were partially reduced in the following manner. A 20 cc stainless steel tube reactor was packed with catalyst, and hydrogen gas was introduced into the reactor at 150—200 cc/min at atmospheric pressure. The electric block furnace placed around the reactor was increased in 50° increments stepwise until 500°C was reached. The final temperature was maintained for two hours, at which time the reactor was allowed to cool with hydrogen flow being continued.

Certain catalysts identified below were nitrided after reduction by contacting the catalyst with ammonia for several hours at atmospheric pressure and a temperature of about 400°C, with subsequent cooling under ammonia. The nitrided catalysts contained up to 1 weight % nitrogen, as is preferred. Between 0.5 and 1 weight percent nitrogen is most preferred.

Reaction procedure

Following catalyst reduction (and nitriding if applicable) and subsequent cooling to room temperature, the reactor was charged to the desired pressure with hydrogen. The split block electric furnace surrounding the reactor was activated and set for run temperature. The system was allowed to equilibrate for at least 15 minutes at run temperature before carbon monoxide flow was started and both gases were adjusted to the desired flow rate. After about one to one and one-half hours of reaction, the off-gas (effluent) was sampled and analyzed and the condensible product diverted from a pre-run receiver to a product collection receiver.

A recovery run proceeded for one to three hours during which time the off-gas was analyzed by gas chromatography and its volume measured. The liquid product also was weighed and analyzed.

In addition to gas chromatography analysis for the gas phase, hydrocarbons having more than three carbon atoms were determined by flame ionization detection. Liquid phase hydrocarbons and oxygenated hydrocarbons were analyzed by gas chromatography. The results reported in the Tables below were calculated as follows.

$$\text{Selectivity}=\frac{\text{Moles product}\times\text{number of carbon atoms in product}\times 100}{\text{Moles CO input}-\text{Moles Co effluent}}$$

$$\text{CO Conversion}=\frac{\text{Moles of CO input}-\text{moles CO effluent}\times 100}{\text{Moles of CO input}}$$

Selectivity to gas and aqueous phase products are reported as a percent of total products. Selectivity to oil phase products are reported as a percent of total oil phase product obtained, calculated as above. Weight percent hydrocarbons are reported as weight percent of total product weight. Carbon dioxide and water are not considered in the calculations.

The catalysts identified in the examples below were prepared according to the catalyst preparation methods set forth above. The catalysts were reduced, and where identified were nitrided, and tested for synthesis gas upgrading by the reaction procedure set forth above. Reaction conditions and test results are set forth in the Tables below.

Examples 1—2

Catalysts of the formula 5% $Na_bRuCuO_x$/95% Alundum were prepared according to the procedure first set forth above. Products of the synthesis gas upgrading reaction utilizing these catalysts were predominantly olefins and carboxylic acids.

Examples 3—4

Catalysts of formula 5% $Na_bRuCu_2O_x$/95% Alundum were prepared according to the procedure of Example 1 except that twice the level of copper chloride was utilized in the catalyst preparation. Product selectivity to olefins and paraffins increased, with good selectivity to carboxylic acids remaining.

Example 5

The catalyst 5% $Na_bRuCuN_zO_x$/95% Alundum was prepared by nitriding the catalyst of Example 1 by the nitriding procedure set forth above. The catalyst continued to show selectivity to olefins and carboxylic acids, with an increase in alcohol production.

Example 6

A catalyst of the formula 5% $Na_bRuCuO_x$/95% Alundum was prepared according to the procedure of Example 1. The predominant products of synthesis gas upgrading using this catalyst were carboxylic acids and olefins.

Examples 7—14

Catalysts of the formula 5% $Na_bRuCuN_zO_x$/95% Alundum were prepared according to the procedure of Example 5. Predominant products again were olefins and carboxylic acids.

Examples 15—23

The promoter metals listed below were added by coprecipitation or in the initial catalyst component slurry, or were added by impregnating the $RuCuO_x$ containing powder to form 5% $M_aNa_{0.02-0.2}RuCuN_zO_x$/95% Alundum catalysts by the method first set forth above, with nitriding. The catalysts were tested for synthesis gas upgrading under the conditions listed in Table III. These catalysts also exhibit good selectivity to olefins and carboxylic acids.

| Example No. | Promoter ($M_a$) | Compound | Addition |
|---|---|---|---|
| 15 | $Ce_{0.2}$ | cerium oxide | initial slurry |
| 16 | $Cr_{0.2}$ | chromium chloride | coprecipitation |
| 17 | $Fe_{0.1}$ | iron chloride | coprecipitation |
| 18 | $Mn_{0.2}$ | manganese chloride | coprecipitation |
| 19 | $Mn_{0.2}$ | manganese chloride | coprecipitation |
| 20 | $Mo_{0.1}$ | ammonium heptamolybdate | coprecipitation |
| 21 | $Mo_{0.1}$ | ammonium heptamolybdate | coprecipitation |
| 22 | $Zn_{0.5}$ | zinc nitrate | impregnation |

Comparative Examples 23—24

Alkali metal/alkaline earth metal-free catalysts of the formula 5% $RuCuN_zO_x$/95% Alundum were prepared from a solution of ruthenium nitrate and copper nitrate, with nitriding of the catalyst being conducted after partial reduction. The catalysts were tested for synthesis gas upgrading under the conditions listed in Table IV, resulting in predominantly methane production.

Examples 25—34

The alkali and alkaline earth metals listed below were added to form the catalysts listed in Table IV by impregnation of either $RuCuO_x$ or $RuCuNa_{0.3}O_x$ catalysts, prepared with nitriding. These catalysts exhibit good selectivity to olefins and carboxylic acids as reported in Table IV.

| Example No. | $A_b$ Promoter added | Compound added | Base catalyst |
|---|---|---|---|
| 25 | $Na_{0.2}$ | sodium carbonate | $RuCuNa_{0.3}O_x$ |
| 26 | $Li_{0.2}$ | lithium hydroxide | $RuCuNa_{0.3}O_x$ |
| 27 | $Li_{0.2}$ | lithium hydroxide | $RuCuNa_{0.3}O_x$ |
| 28 | $Mg_{0.2}$ | magnesium hydroxide | $RuCuNa_{0.3}O_x$ |
| 29 | $Mg_{0.2}$ | magnesium hydroxide | $RuCuNa_{0.3}O_x$ |
| 30 | $Na_{0.2}$ | sodium carbonate | $RuCuO_x$ |
| 31 | $Na_{0.2}$ | sodium carbonate | $RuCuNa_{0.3}O_x$ |
| 32 | $Cs_{0.2}$ | cesium acetate | $RuCuO_x$ |
| 33 | $Li_{0.4}$ | lithium hydroxide | $RuCuO_x$ |
| 34 | $Li_{0.4}$ | lithium hydroxide | $RuCuO_x$ |

Example 35

A catalyst of the formula 5% $Na_{0.02-0.2}RuCuN_z$/95% $SiO_2$ was prepared according to the method of Example 5 except that silica was substituted for the alumina silica-Alundum support. The catalyst was tested for synthesis gas upgrading at a temperature of 350°C, a pressure of 1300 psi, (8.963 MPa) a space velocity of 330 per hour and a Co:$H_2$ ratio of 3:7. Co conversion was 31.2% and the product distribution was as follows:

| Product | Wt.% |
|---|---|
| Alkanes | 28.9 |
| Olefins | 30.5 |
| Carboxylic acid | 20.3 |
| Alcohols | 13.0 |
| Aldehydes | 1.8 |
| Esters | 5.4 |

This catalyst exhibited good selectivity to olefins and carboxylic acids.

As an example of the product mixture produced by the inventive process, the products obtained testing the catalyst of Example 33 are as follows.

| Product | Wt. (g) |
|---|---|
| Methane | 0.2832 |
| Ethane | 0.0500 |
| Propane | 0.1393 |
| Ethylene | 0.0681 |
| Propylene | 0.2850 |
| Methanol | 0.0278 |
| Ethanol | 0.0291 |
| Propanol | 0.0093 |
| Butanol | 0.0111 |
| Pentanol | 0.0043 |
| Acetic acid | 0.0585 |
| Propionic acid | 0.0369 |
| Butyric acid | 0.0202 |
| Valeric acid | 0.0097 |
| Higher alkanes | 0.0528 |
| Higher olefins | 0.2097 |
| Higher alcohols | 0.1656 |
| Higher acids | 0.3635 |
| Aldehydes | 0.0501 |
| Esters | 0.0383 |

Example 36

A portion of the liquid products of the process of the present invention, comprising mainly carboxylic acids and olefins, with minor amounts of alcohols and aldehydes, was hydrogenated in the vapour phase at a reaction temperature of 200°C and a pressure of 1000 psi (6.894 MPa) in the presence of a hydrogenation catalyst comprising 5% $RuCoPdZn_{0.4}O_x$ on 95% Alundum. Hydrogen was introduced to the reaction at 300 cc/minute, and hydrocarbon liquid was introduced to the reaction at 5 cc/hr. Olefins and aldehydes were completely converted to alkanes and alcohols, and over 90% of the acids were converted to either alcohols or esters. The hydrogenated products of the process of the present invention, alkanes, alcohols and esters, are useful for fuels.

TABLE I
Synthesis gas upgrading using promoted 5% $RuCuO_x$/95% Alundum catalysts

| Ex. No. | Catalyst | Temp. °C | Pressure (psi) (MPa) | Space vel. ($hr^{-1}$) | $CO:H_2$ ratio | % CO conversion | Product phase, wt.% (a) | | |
|---|---|---|---|---|---|---|---|---|---|
| | | | | | | | Gas | Aqueous | Organic |
| 1 | $Na_bRuCuO_x$ | 350 | 1300/8.963 | 3300 | 3:7 | 42.2 | 45.5 | 17.8 | 36.7 |
| 2 | $Na_bRuCuO_x$ | 350 | 600/6.133 | 510 | 1:1 | 71.4 | 18.7 | 12.9 | 68.4 |
| 3 | $Na_bRuCu_2O_x$ | 350 | 1300/8.963 | 3300 | 3:7 | 79.4 | 41.8 | 9.6 | 48.7 |
| 4 | $Na_bRuCu_2O_x$ | 350 | 600/6.133 | 510 | 1:1 | 98.1 | 30.1 | 2.3 | 67.5 |
| 5 | $Na_bRuCuN_zO_x$ | 350 | 1300/8.963 | 3300 | 3:7 | 31.2 | 56.4 | 12.5 | 31.1 |
| 6 | $Na_bRuCuO_x$ | 350 | 1300/8.963 | 3300 | 3:7 | 35.6 | 34.2 | 14.8 | 51.0 |
| 7 | $Na_bRuCuN_zO_x$ | 350 | 1300/8.963 | 3300 | 3:7 | 34.9 | 38.0 | 19.9 | 42.1 |
| 8 | $Na_bRuCuN_zO_x$ | 350 | 1300/8.963 | 3300 | 3:7 | 32.4 | 33.4 | 16.5 | 50.1 |
| 9 | $Na_bRuCuN_zO_x$ | 350 | 1300/8.963 | 3300 | 3:7 | 59.1 | 43.7 | 9.5 | 46.8 |
| 10 | $Na_bRuCuN_zO_x$ | 340 | 1300/8.963 | 3300 | 3:7 | 35.4 | 25.7 | 13.3 | 61.0 |
| 11 | $Na_bRuCuN_zO_x$ | 340 | 1300/8.963 | 3300 | 3:7 | 35.6 | 33.8 | 14.2 | 52.0 |
| 12 | $Na_bRuCuN_zO_x$ | 340 | 1300/8.963 | 3300 | 3:7 | 31.8 | 24.3 | 12.9 | 62.7 |
| 13 | $Na_bRuCuN_zO_x$ | 320 | 1000/6.894 | 5500 | 3:7 | 4.0 | 18.9 | 24.3 | 56.8 |
| 14 | $Na_bRuCuN_zO_x$ | 345 | 1300/8.963 | 3300 | 3:7 | 39.4 | 48.2 | 9.9 | 41.9 |

(a) excludes weight of $H_2O$ and $CO_2$
b=0.02 to 0.2
z=0.5—1 wt.% active catalyst

TABLE II
Synthesis gas upgrading

| Ex. No. | % Selectivity (total) gas phase | | | % Selectivity (total) aqueous phase | | % Selectivity organic phase | | | | | Wt.% paraffin |
|---|---|---|---|---|---|---|---|---|---|---|---|
| | $CH_4$ | Alkanes $\geq C_2$ | Olefins | Alcohol | Carboxylic acid | Alcohol | Carboxylic acid | Ester | Aldehyde | Olefin | |
| 1 | 9.9 | 5.4 | 16.4 | 1.3 | 5.6 | 17.1 | 42.2 | — | 15.6 | 25.0 | tr |
| 2 | 1.8 | 0.8 | 3.3 | 0.4 | 1.8 | 20.1 | 48.5 | — | 5.3 | 26.1 | — |
| 3 | 10.4 | 5.3 | 13.6 | 1.4 | 2.4 | 18.4 | 28.7 | 3.0 | 3.2 | 46.7 | 33 |
| 4 | 4.3 | 4.2 | 14.4 | 0.6 | 0.3 | 20.1 | 25.0 | 9.6 | 2.1 | 43.2 | 28 |
| 5 | 12.0 | 8.3 | 32.3 | 1.9 | 4.8 | 33.7 | 39.4 | 1.1 | 9.7 | 16.2 | tr |
| 6 | 7.3 | 5.6 | 15.4 | 1.3 | 4.2 | 16.7 | 43.7 | 2.8 | 8.5 | 28.3 | 4 |
| 7 | 4.4 | 5.9 | 9.9 | 1.0 | 3.6 | 15.9 | 52.5 | 1.3 | 3.6 | 26.7 | 5 |
| 8 | 7.7 | 6.4 | 11.9 | 1.3 | 3.9 | 15.3 | 44.1 | 2.5 | 7.4 | 30.8 | — |
| 9 | 11.1 | 6.4 | 13.3 | 1.3 | 4.8 | 17.9 | 35.2 | 2.3 | 7.1 | 37.5 | 5 |
| 10 | 3.6 | 5.6 | 9.3 | 1.1 | 4.2 | 16.5 | 43.4 | 2.6 | 11.2 | 26.3 | 2 |
| 11 | 7.2 | 4.4 | 8.2 | 0.9 | 3.7 | 13.1 | 44.1 | 2.1 | 12.0 | 28.8 | 2 |
| 12 | 4.8 | 5.2 | 10.3 | 0.5 | 4.6 | 13.4 | 40.0 | 2.1 | 11.6 | 32.9 | 2 |
| 13 | 8.2 | — | 3.2 | 0.5 | 7.5 | 7.7 | 69.3 | 4.0 | 10.8 | 8.2 | — |
| 14 | 3.7 | 9.0 | 28.2 | 0.9 | 3.3 | 12.8 | 49.2 | 3.0 | 4.7 | 30.4 | 2 |

*Trace aldehydes present

TABLE III

Synthesis gas upgrading using 5% $M_aNa_bCuRuN_zO_x$/95% Alundum catalysts

| Ex. No. | $M_a$ promoter | Temp. (°C) | Pressure (psig) (MPa) | Space velocity ($hr^{-1}$) | % CO conv. | Weight % useful products | | | | | |
|---|---|---|---|---|---|---|---|---|---|---|---|
| | | | | | | Alkanes | Olefins | Carboxylic acids | Alcohols | Aldehydes | Esters |
| 15 | $Ce_{0.2}$ | 340 | 1300/8.963 | 3300 | 92.4 | 51.2 | 24.2 | 13.3 | 9.4 | 0.4 | 1.5 |
| 16 | $Cr_{0.2}$ | 350 | 1300/8.963 | 3300 | 82.9 | 51.5 | 24.7 | 14.9 | 7.6 | 0.4 | 0.9 |
| 17 | $Fe_{0.1}$ | 300 | 1000/6.894 | 5500 | 9.5 | 45.0 | 14.9 | 18.5 | 9.6 | 3.9 | 1.3 |
| 18 | $Mn_{0.2}$ | 310 | 1000/6.894 | 5500 | 10.9 | 30.9 | 28.9 | 24.1 | 6.8 | 7.2 | 2.0 |
| 19 | $Mn_{0.2}$ | 315 | 1300/8.963 | 3300 | 60.4 | 41.1 | 29.7 | 16.7 | 7.0 | 2.9 | 2.5 |
| 20 | $Mo_{0.1}$ | 320 | 1000/6.894 | 5500 | 15.1 | 61.4 | 23.2 | 9.0 | 8.3 | — | — |
| 21 | $Mo_{0.1}$ | 350 | 1000/6.894 | 5500 | 40.6 | 47.4 | 22.8 | 15.1 | 13.8 | 0.8 | — |
| 22 | $Zn_{0.5}$ | 340 | 1300/8.963 | 3300 | 4.4 | 48.7 | 34.7 | 4.3 | 12.3 | — | — |

b=0.02—0.2
z=0.5—1 wt.% active catalyst
$CO:H_2$ ratio=3:7

TABLE IV

Synthesis gas upgrading using 5% $A_bRuCuN_zO_x$/95% Alundum catalysts

| Ex. No. | $A_b$ promoter | Temp. (°C) | Pressure (psig) (MPa) | Space velocity ($hr^{-1}$) | % CO conversion | Weight % useful products | | | | | |
|---|---|---|---|---|---|---|---|---|---|---|---|
| | | | | | | Alkanes | Olefins | Carboxylic acids | Alcohols | Aldehydes | Esters |
| C23 | — | 360 | 1300/8.963 | 3300 | tr | 100 | — | — | — | — | — |
| C24 | — | 320 | 1000/6.894 | 5500 | tr | 70.9 | — | 15 | 14.1 | — | — |
| 25 | $Na_{0.5}$ | 320 | 1000/6.894 | 5500 | 6.6 | 12.5 | 15.8 | 49.2 | 11.4 | 7.6 | 3.5 |
| 26 | $Li_{0.2}Na_{0.3}$ | 320 | 1000/6.894 | 5500 | 13.8 | 20 | 22.3 | 46.0 | 9.2 | 7.1 | 5.3 |
| 27 | $Li_{0.2}Na_{0.3}$ | 340 | 1300/8.963 | 3300 | 42.4 | 25.5 | 34.2 | 25.0 | 9.9 | 2.7 | 2.6 |
| 28 | $Mg_{0.2}Na_{0.3}$ | 320 | 1000/6.894 | 5500 | 15.3 | 27.3 | 34.2 | 21.5 | 8.5 | 6.2 | 2.3 |
| 29 | $Mg_{0.2}Na_{0.3}$ | 330 | 1300/8.963 | 3300 | 77.0 | 38.0 | 31.6 | 16.0 | 8.5 | 1.2 | 1.7 |
| 30 | $Na_{0.2}$ | 350 | 1300/8.963 | 3300 | 35.5 | 29.4 | 29.2 | 25.0 | 11.2 | 3.0 | 2.3 |
| 31 | $Na_{0.5}$ | 340 | 1300/8.963 | 3300 | 46.1 | 24.5 | 30.6 | 27.0 | 11.7 | 4.0 | 2.3 |
| 32 | $Cs_{0.2}$ | 350 | 1300/8.963 | 3300 | 17.0 | 72.8 | 8.1 | 12.4 | 6.7 | — | — |
| 33 | $Li_{0.4}$ | 320 | 1000/6.894 | 5500 | 13.2 | 27.5 | 29.4 | 25.6 | 12.9 | 2.6 | 2.0 |
| 34 | $Li_{0.4}$ | 330 | 1300/8.963 | 3300 | 43.5 | 34.4 | 35.1 | 15.8 | 10.6 | 2.1 | 2.0 |

**Claims**

1. A catalyst composition of the formula

$$M_aA_bRuCu_cN_zO_x$$

wherein A is an alkali metal or an alkaline earth metal or a mixture thereof,
wherein M is Ce, Cr, Fe, Mn, Mo, Zn or mixtures thereof, and
wherein a is 0 to 0.5,
b is 0.002 to 2,
c is 0.5 to 3,
z is 0 to 1 weight percent and
x is the number of oxygens needed to fulfill the valence requirements of the other elements.

2. A catalyst as claimed in claim 1 characterised in that A is selected from Na, Li, K, Rb, Cs, Mg or mixtures thereof.

3. A catalyst as claimed in claim 1 or claim 2 characterised in that a is 0.1 to 0.5.

4. A catalyst as claimed in any of claims 1 to 3 characterised in that b is 0.02 to 1.

5. A catalyst as claimed in any of claims 1 to 4 characterised in that c is 1.

6. A catalyst as claimed in any of claims 1 to 5 characterised in that the catalyst is partially reduced.

7. A catalyst as claimed in any of claims 1 to 6 characterised in that the catalyst is supported on an inert carrier, preferably alumina, silica, alumina-silica, clay or silicon carbide.

8. A process for the upgrading of synthesis gas to obtain selectivity to olefins and carboxylic acids comprising contacting carbon monoxide and hydrogen in the vapour phase at a reaction temperature of at least 250°C and a reaction pressure of at least 500 psi (3.447 MPa) with a catalyst as claimed in any of claims 1 to 7.

9. A process as claimed in claim 8 characterised in that the ratio of carbon monoxide to hydrogen is 10:1 to 1:10, preferably 3:1 to 1:3.

10. A process as claimed in claim 9 characterised in that the reaction temperature is from 275 to 375°C.

11. A process for the upgrading of synthesis gas to yield hydrocarbons, alcohols and esters useful for fuel, comprising contacting carbon monoxide and hydrogen in the vapour phase as claimed in any of claims 8 to 10;
recovering the resulting hydrocarbon and oxygenated hydrocarbon products; and
contacting said products with hydrogen at elevated temperature and pressure in the presence of a hydrogenation catalyst.

12. A process as claimed in claim 11 characterised in that the products are contacted with hydrogen at a temperature of at least 200°C.

13. A process as claimed in claim 11 or claim 12 characterised in that the products are contacted with hydrogen at a pressure of 500 psi to 5,000 psi (3.447 to 34.47 MPa).

14. A process as claimed in any of claims 11 to 13 characterised in that the hydrogenation catalyst is represented by the formula

$$G_eRu_fD_gE_hO_x$$

wherein G=Zn, Cd and mixtures thereof;
D=Co, Ni and mixtures thereof;
E=Fe, Cu, Rh, Pd, Os, Ir, Pt and mixtures thereof; and
wherein e=0 to 1;
f=0.01 to 3,
g=0.01 to 3,
h=0 to 1,
x=the number of oxygens determined by the valence requirements of the other elements.

15. A process as claimed in claim 14 characterised in that the hydrogenation catalyst has the formula:

$$RuCoPdZn_{0.4}O_x.$$

16. A process for the upgrading of synthesis gas to yield hydrocarbons, alcohols and esters useful for fuel, comprising contacting carbon monoxide and hydrogen in the vapour phase at a reaction temperature of at least 250°C and a reaction pressure of at least 500 psi (3.447 MPa) with a catalyst of the formula:

$$M_aA_bRuCu_cN_zO_x$$

wherein A is an alkali metal or an alkaline earth metal or a mixture thereof,
wherein M is Ce, Cr, Fe, Mn, Mo, Zn or mixtures thereof, and
wherein a is 0 to 0,5,
b is 0.002 to 2,
c is 0.5 to 3,

z is 0 to 1 weight percent, and

x is the number of oxygens needed to fulfill the valence requirements of the other elements;

recovering the resulting hydrocarbon and oxygenated hydrocarbon products; and

contacting said products with hydrogen at elevated temperature and pressure in the presence of a hydrogenation catalyst represented by the formula:

$$G_eRu_fD_gE_hO_x$$

wherein G=Zn, Cd and mixtures thereof;
D=Co, Ni and mixtures thereof;
E=Fe, Cu, Rh, Pd, Os, Ir, Pt and mixtures thereof; and
wherein e=0 to 1;
f=0.01 to 3,
g=0.01 to 3,
h=0 to 1,
x=the number of oxygens determined by the valence requirements of the other elements.

**Patentansprüche**

1. Eine Katalysator-Zusammensetzung der Formel

$$M_aA_bRuCu_cN_zO_x$$

wobei A ein Alkalimetall oder ein Erdalkalimetall oder eine Mischung davon ist,
worin M Ce, Cr, Fe, Mn, Mo, Zn oder Mischungen davon ist, und
worin a 0 bis 0,5 ist,
b 0,002 bis 2 ist,
c 0,5 bis 3 ist,
z 0 bis 1 Gew.-% und
x die Zahl der Sauerstoffe ist, die notwendig sind, um die Wertigkeitsanforderungen der anderen Elemente erfüllen.

2. Katalysator nach Anspruch 1, dadurch gekennzeichnet, daß A aus Na, Li, K, Rb, Cs, Mg oder Mischungen davon ausgewählt wird.

3. Katalysator nach Anspruch 1 oder 2, dadurch gekennzeichnet, daß a 0,1 bis 0,5 ist.

4. Katalysator nach einem der Ansprüche 1 bis 3, dadurch gekennzeichnet, daß b 0,02 bis 1 ist.

5. Katalysator nach einem der Ansprüche 1 bis 4, dadurch gekennzeichnet, daß c 1 ist.

6. Katalysator nach einem der Ansprüche 1 bis 5, dadurch gekennzeichnet, daß der Katalysator teilweise reduziert ist.

7. Katalysator nach einem der Ansprüche 1 bis 6, dadurch gekennzeichnet, daß der Katalysator auf einem inerten Trägermaterial, vorzugsweise Aluminiumoxid, Siliciumoxid, Aluminiumoxid-Siliciumoxid, Tonerde oder Siliciumcarbid aufgetragen ist.

8. Verfahren zur Veredelung von Synthesegas, wobei man Selektivität in Bezug auf Olefine und Carbonsäuren erhält, das umfaßt in Kontaktbringen von Kohlenmonoxid und Wasserstoff in der Gasphase bei einer Reaktionstemperatur von wenigstens 250°C und einem Reaktionsdruck von wenigstens 500 psi (3,447 MPa) mit einem Katalysator nach einem der Ansprüche 1 bis 7.

9. Verfahren nach Anspruch 8, dadurch gekennzeichnet, daß das Verhältnis von Kohlenmonoxid zu Wasserstoff 10:1 bis 1:10, bevorzugt 3:1 bis 1:3 beträgt.

10. Verfahren nach Anspruch 9, dadurch gekennzeichnet, daß die Reaktionstemperatur von 275° bis 375°C reicht.

11. Verfahren zur Veredelung von Synthesegas, wobei man Kohlenwasserstoffe, Alkohole und Ester, die als Brennstoff verwendbar sind, erhält, das umfaßt Inkontaktbringen von Kohlenmonoxid und Wasserstoff in der Gasphase nach einem der Ansprüche 8 bis 10;

Gewinnung der sich ergebenden Kohlenwasserstoff- und oxidierten Kohlenwasserstoffprodukte; und

in Kontaktbringen dieser Produkte mit Wasserstoff bei erhöhter Temperatur und Druck in Gegenwart eines Hydrierkatalysators.

12. Verfahren nach Anspruch 11, dadurch gekennzeichnet, daß die Produkte mit Wasserstoff bei einer Temperatur von wenigstnes 200°C in Kontakt gebracht werden.

13. Verfahren nach Anspruch 11 oder 12, dadurch gekennzeichnet, daß die Produkte mit Wasserstoff bei einem Druck von 500 psi bis 5000 psi (3,447 bis 34,47 MPa) in Kontakt gebracht werden.

14. Verfahren nach einem der Ansprüche 11 bis 13, dadurch gekennzeichnet, daß der Hydrierkatalysator durch die Formel dargestellt wird

$$G_3Ru_fD_gE_hO_x$$

worin G=Zn, Cd und Mischungen davon ist,
D=Co, Ni und Mischungen davon ist;
E=Fe, Cu, Rh, Pd, Ps, Ir, Pt und Mischungen davon ist; und

worin e=0 bis 1;
f=0.01 bis 3,
g=0.01 bis 3,
h=0 bis 1,
x=der Anzahl der Sauerstoffe ist, festgelegt durch die Wertigkeitsanforderungen der anderen Elemente.

15. Verfahren nach Anspruch 14, dadurch gekennzeichnet, daß der Hydrierkatalysator die Formel hat:

$$RuCoPdZn_{0.4}O_x$$

16. Verfahren zum Veredeln von Synthesegas, wobei man Kohlenwasserstoffe, Alkohole und Ester, die als Brennstoff verwendbar sind, erhält, das umfaßt Inkontaktbringen von Kohlenmonoxid und Wasserstoff in der Gasphase bei einer Reaktonstemperatur von wenigstens 250°C unter einem Reaktionsdruck von wenigstens 500 psi (3,447 MPa) mit einem Katalysator der Formel:

$$M_aA_bRuCu_cN_zO_x$$

worin A ein Alkalimetall oder Erdalkalimetall oder eine Mischung davon ist,
worin M Ce, Cr, Fe, Mn, Mo, Zn oder Mischungen davon ist, und
worin a 0 bis 0,5 ist,
b 0,002 bis 2 ist,
c 0,5 bis 3 ist,
z 0 bis 1 Gew.% ist und
x die Anzahl der Sauerstoffatome ist, die benötigt werden, um die Wertigkeitsanforderungen der anderen Elemente zu erfüllen;

Gewinnung der sich ergebenden Kohlenwasserstoff- und oxidierten Kohlenwasserstoffprodukte; und in Kontaktbringen dieser Produkte mit Wasserstoff bei erhöhter Temperatur und Druck in Gegenwart eines Hydrierkatalysators, dargestellt durch die Formel:

$$G_eRu_fD_gE_hO_x$$

worin G=Zn, Cd und Mischungen davon;
D=Co, Ni und Mischungen davon
E=Fe, Cu, Rh, Pd, Os, Ir, Pt und Mischungen davon sind; und
worin e=0 bis 1;
f=0,01 bis 3,
g=0,01 bis 3,
h=0 bis 1,
x=der Anzahl der Sauerstoffatome ist, festgelegt durch die Wertigkeitsanforderungen der anderen Elemente.

**Revendications**

1. Composition du catalyseur de la formule:

$$M_aA_bRuCu_cN_zO_x$$

où A est un métal alcalin ou un métal alcalino-terreux ou un mélange de ces métaux,
où M est Ce, Cr, Fe, Mn, Mo, Zn ou des mélanges de ces corps, et
où a est 0 à 0.5,
b est 0.002 à 2,
c est 0.5 à 3,
z est 0 à 1 pourcent en poids et
x est le nombre d'oxygènes nécessaire pour remplir les conditions de valence des autres éléments.

2. Catalyseur selon la revendication 1 caractérisé en ce que l'on choisit A parmi Na, Li, K, Rb, Cs, Mg ou leurs mélanges.

3. Catalyseur selon la revendication 1 ou la revendication 2, caractérisé en ce que a est 0.1 à 0.5.

4. Catalyseur selon l'une quelconque des revendications 1 à 3, caractérisé en ce que b est 0.02 à 1.

5. Catalyseur selon l'une quelconque des revendications 1 à 4, caractérisé en ce que c est 1.

6. Catalyseur selon l'une quelconque des revendications 1 à 5, caractérisé en ce que le catalyseur est partiellement réduit.

7. Catalyseur selon l'une quelconque des revendications 1 à 6, caractérisé en ce que le catalyseur est supporté sur un porteur inerte, de préférence de l'alumine, de la silice, de l'alumine-silice, de l'argile ou du carbure de silicium.

8. Procédé pour la valorisation du gaz de synthèse pour obtenir une sélectivité concernant les oléfines

et les acides carboxyliques, comportant le fait de mettre en contact de l'oxyde de carbone et de l'hydrogène en phase vapeur à une température de réaction d'au moins 250°C et sous une pression de réaction d'au moins 500 psi (3.447 MPa) avec un catalyseur tel que revendiqué dans l'une quelconque des revendications 1 à 7.

9. Procédé selon la revendication 8, caractérisé en ce que le rapport de l'oxyde de carbone à l'hydrogène est 10:1 à 1:10, de préférence 3:1 à 1:3.

10. Procédé selon la revendication 9, caractérisé en ce que la température de la réaction est située entre 275 et 375°C.

11. Procédé pour la valorisation du gaz de synthèse pour donner des carbures d'hydrogène, des alcools et des esters utilisés comme carburants, comportant les étapes de mettre en contact l'oxyde de carbone et l'hydrogène en phase vapeur comme revendiqué dans l'une quelconque des revendications 8 à 10;

de récupérer les produits hydrocarbonés et les produits hydrocarbonés oxygénés résultants; et

de mettre en contact lesdits produits avec de l'hydrogène à température élevée et sous pression élevée en présence d'un catalyseur d'hydrogénation.

12. Procédé selon la revendication 11, caractérisé en ce que l'on met les produits en contact avec l'hydrogène sous une température d'au moins 200°C.

13. Procédé selon la revendication 11 ou de la revendication 12, caractérisé en ce que l'on met les produits en contact avec l'hydrogène sous une pression de 500 psi à 5.000 psi (3.447 à 34.47 MPa).

14. Procédé selon l'une quelconque des revendications 11 à 13, caractérisé en ce que le catalyseur d'hydrogénation est représenté par la formule:

$$G_eRu_fD_gE_hO_x$$

où G=Zn, Cd et leurs mélanges,
D=Co, Ni et leurs mélanges,
E=Fe, Cu, Rh, Pd, Os, Ir, Pt et leurs mélanges, et
où e=0 à 1,
f=0.01 à 3,
g=0.01 à 3,
h=0 à 1,
x=le nombre d'oxygènes déterminé par les conditions de valence des autres éléments.

15. Procédé selon la revendication 14, caractérisé en ce que le catalyseur d'hydrogénation à la formule:

$$RuCoPdZn_{0.4}O_x$$

16. Procédé pour la valorisation du gaz de synthèse pour donner des carbures d'hydrogène, des alcools et des esters utilisés comme carburants, comportant les étapes de mettre en contact l'oxyde de carbone et l'hydrogène en phase vapeur à une température de réaction d'au moins 250°C et sous une pression de réaction de au moins 500 psi (3.447 MPa) avec un catalyseur de la formule:

$$M_aA_bRuCu_cN_zO_x$$

où A est un métal alcalin ou un métal alcalino-terreux ou un mélange de ces métaux,
où M est Ce, Cr, Fe, Mn, Mo, Zn ou des mélanges de ces corps, et
où a est 0 à 0.5,
b est 0.002 à 2,
c est 0.5 à 3,
z est 0 à 1 pourcent en poids, et
x est le nombre d'oxygènes nécessaire pour remplir les conditions de valence des autres éléments;

de récupérer les produits hydrocarbonés et les produits hydrocarbonés oxygénés résultants; et

mettre en contact lesdits produits avec de l'hydrogène à température élevée et sous une pression élevée en présence d'un catalyseur d'hydrogénation représenté par la formule:

$$G_eRu_fD_gE_hO_x$$

où G=Zn, Cd et leurs mélanges,
D=Co, Ni et leurs mélanges,
E=Fe, Cu, Rh, Pd, Os, Ir, Pt et leurs mélanges, et
où e=0 à 1,
f=0.01 à 3,
g=0.01 à 3,
h=0 à 1,
x=le nombre d'oxygènes déterminé par les conditions de valence des autres éléments.